# EUROPÄISCHE PATENTSCHRIFT

(11) **EP 2 085 153 B1**
(45) Veröffentlichungstag und Bekanntmachung des Hinweises auf die Patenterteilung: **07.03.2012**
(21) Anmeldenummer: 09002147.8
(22) Anmeldetag: 05.07.2007
(51) Int. Cl.: B07C 5/34, G01N 3/48

(54) **Vorrichtung und Verfahren zur Klassifikation auftreffender Körper durch Erfassen des zeitlichen Beschleunigungsverlaufs, sowie eine solche Vorrichtung umfassende Entkernvorrichtung**
Device and method for classification of impacting bodies by detection of acceleration progress over time and fruit pitting machine comprising such a device
Dispositif et procédé de classification de corps d'incidence par détection d'écoulement d'accélération et machine pour dénoyauter des fruits avec un tel dispositif

(30) Priorität: 22.08.2006 DE 102006039390
(43) Veröffentlichungstag der Anmeldung: 05.08.2009
(62) Teilanmeldung aus: 07765096.8
(73) Patentinhaber: Fraunhofer-Gesellschaft zur Förderung der angewandten Forschung e.V., 80686 München (DE)
(72) Erfinder: Schmitt, Peter, 91058 Erlangen (DE); Scholz, Oliver, 91096 Moehrendorf (DE); Kostka, Guenther, 91056 Erlangen (DE)
(74) Vertreter: Schenk, Markus

(56) Entgegenhaltungen:
- WO-A-03/045590
- US-A- 3 127 016

## Beschreibung

Die vorliegende Erfindung bezieht sich auf die Klassifikation auftreffender Körper und insbesondere, wie ein auf einen Auftreffkörper auftreffender Körper beispielsweise bezüglich seiner Materialeigenschaften oder seines Materialtyps klassifiziert werden kann (siehe US-A-3,127,016 oder WO-A-03/045590).

Eine industriell besonders interessante Anwendungsform einer solchen Klassifikation auftreffender Körper ist die Erkennung von Kernen, die aus Steinobstfrüchten entfernt wurden und auf den Auftreffkörper treffen.

Eine zuverlässige Entkernung von Steinobstfrüchten wie Kirschen oder Pflaumen im industriellen Maßstab ist wichtig, da bei einem unvollständig entfernten Kern bzw. Stein einem Konsumenten beim Verzehr von Steinobst Verletzungen im Zahnbereich entstehen können.

Bei den im industriellen Maßstab üblichen Entkernverfahren werden die Kerne mechanisch aus der Steinobstfrucht entfernt, wobei wie etwa bei Haushaltskirschentkernern üblich, das zu entsteinende Obst von einem Stößel durchstoßen und der Stein dabei aus der Frucht befördert wird. Industriell werden dazu zunächst mehrere Steinobstfrüchte in diskreten Portionen variabler Größe in eine Haltvorrichtung einsortiert. Die Haltvorrichtung, z.B. in Form einer Walze, ist dabei so beschaffen, dass sie die Früchte in definierten, diskreten Positionen aufnimmt. Beim eigentlichen Entkernvorgang werden die Früchte mechanisch durch einen beispielsweise pneumatisch angetriebenen Stößel durchstoßen, wobei der Kern der Frucht auf der dem Stößeleintrittspunkt gegenüberliegenden Seite aus der Frucht entfernt wird. Probleme entstehen, wenn der Kern vom Stößel nicht zentral getroffen wird, denn dann kann es passieren, dass der Kern entweder nicht aus der Frucht entfernt wird oder durch den dezentralen Stoß des Stößels der Kern gespalten und nur Teile des Kerns aus der Frucht entfernt werden. Aus den oben genannten Gründen ist die Kontrolle, ob der Kern vollständig aus der Frucht entfernt wurde, unabdingbar.

Verwendet werden unter Anderem Kontrollverfahren, bei denen überprüft wird, ob nach dem Entkernvorgang in der Frucht noch Teile des Kernes verbleiben, wobei die Verfahren in zerstörungsfreie Verfahren und Verfahren, bei denen die Frucht mechanisch verändert wird, unterschieden werden können. Ein Beispiel für die letztgenannten taktilen Verfahren wird in der DE4306515A1 beschrieben. Dort wird eine mechanische Überprüfung des Erfolges des Entkernvorganges dadurch durchgeführt, dass Testnadeln in die Frucht getrieben werden, wobei die Eindringtiefe der Nadeln als Kriterium für das Vorhandensein eines Kernes verwendet wird, die Tatsache ausnutzend, dass die Nadeln einen harten Kern nicht durchdringen können.

Da die Früchte bei den taktilen Verfahren mechanisch verändert oder sogar teilweise zerstört werden, sind manchmal zerstörungsfreie Prüfmethoden bevorzugt, wie sie zum Beispiel in der DE2538799A1 vorgeschlagen sind. Dort wird die Frucht nach dem Entkernvorgang mittels Röntgenstrahlen durchleuchtet, um einen eventuell im Fruchtfleisch verbliebenen Kern zu detektieren. Die zerstörungsfreie Prüfung mittels Röntgenstrahlung, bei der die Frucht daraufhin durchleuchtet wird, ob sich der Kern noch in der Frucht befindet, hat den Nachteil, dass in der Regel eine sichere Detektion im Obst verbliebener Kerne innerhalb eines Produktstromes nicht möglich ist. Der Kern ist innerhalb der Frucht nämlich vollständig von Fruchtfleisch umgeben, womit aufgrund des hohen Wassergehaltes des Fruchtfleisches ein nur geringer Kontrastunterschied in den Röntgenaufnahmen einhergeht und daher ein Kern innerhalb der Frucht nur schwer zu identifizieren ist. Daher können nur bei gefriergetrocknetem und dadurch entwässertem Obst in der Steinobstfrucht verbleibende Kerne sicher erkannt werden. Dies ist beispielsweise bei gefriergetrockneten Kirschen möglich.

Im deutschen Patent 10 2005 018 639 wird ein Verfahren beschrieben, das darauf abzielt, den Entkernprozess mit Röntgentechnik zu beobachten und dadurch auf eine sichere Entsteinung zu schließen. Falls innerhalb eines vorgegebenen Zeitfensters kein Kern beobachtet werden kann, wird angenommen, dass der Kern im Steinobst verblieben ist und eine entsprechende Fehlermeldung wird ausgegeben. Die Verwendung der Röntgentechnik verursacht erhebliche Kosten u.a. für Röntgenquelle, Hochspannungsgenerator und Strahlenschutz.

Verfahren, die wie oben beschriebenes Röntgenverfahren darauf abzielen, den aus der Frucht entfernten Kern beispielsweise von Kirschen oder Pflaumen nachzuweisen, umgehen die meisten der oben beschriebenen Probleme.

Bei solchen Verfahren besteht das Problem allgemein darin, den Kern von alternativ oder zusätzlich aus dem Fruchtkörper entfernten Fleisch unterscheiden zu können, um auf ein erfolgreiches Entkernen einer Steinobstfrucht zu schließen. Dazu ist es wesentlich, den zu untersuchenden Körper zu klassifizieren bzw. physikalische Parameter des Körpers geeignet nachzuweisen.

Die Aufgabenstellung beschränkt sich selbstverständlich nicht auf das Entsteinen von Steinobst. Es sind vielmehr eine Reihe von technischen Anwendungen bekannt, bei denen ein Objekt zuverlässig klassifiziert werden muss. Beispielsweise sei hier die Sortierung von Sojabohnen genannt, bei der die Bohnen in "gute" und "schlechte" Bohnen sortiert werden müssen.

Die Aufgabe der vorliegenden Erfindung besteht darin, eine Vorrichtung und ein Verfahren zum Klassifizieren eines Objekts zu schaffen, die es ermöglichen, effizient und zuverlässig Objekte zu klassifizieren.

Diese Aufgabe wird durch eine Vorrichtung gemäß Patentanspruch 1 oder 21 und ein Verfahren gemäß Patenanspruch 20 gelöst.

Der Kerngedanke der vorliegenden Erfindung besteht dabei darin, dass auf einen Auftreffkörper auftreffende Objekte effizienter und mit höherer Zuverlässigkeit bezüglich ihrer Eigenschaften klassifiziert werden können, wenn der durch das Auftreffen des Objekts am Auftreffkörper hervorgerufene zeitliche Beschleunigungsverlauf ausgewertet wird, um das Objekt zu klassifizieren.

Bei einem Ausführungsbeispiel der vorliegenden Erfindung wird ein Auftreffkörper in einem Objektweg, entlang dessen sich ein zu klassifizierendes Objekt bewegt, angeordnet. Der Auftreffkörper umfasst ferner eine Einrichtung zum Erfassen eines zeitlichen Beschleunigungsverlaufs des Auftreffkörpers, die bei Auftreffen des Objekts den Beschleunigungsverlauf am Auftreffkörper misst.

Durch Messen der Beschleunigung ergibt sich dabei insbesondere der große Vorteil, dass mit höchstmöglicher Zeitauflösung der Impulsübertrag bzw. die Krafteinwirkung auf den Auftreffkörper, der von dem Objekt hervorgerufen wird, bestimmt werden kann.

Gegenüber Verfahren, die beispielsweise die Geschwindigkeit oder die Auslenkung eines von einem Objekt getroffenen Körpers bestimmen, hat dies den großen Vorteil, dass die maximale Information erhalten werden kann, da Geschwindigkeit und Ortsinformation bereits integrale Größen sind, auf die beispielsweise durch Integration des Beschleunigungsverlaufs geschlossen werden kann. Kann, wie erfindungsgemäß, die Beschleunigung zeitlich aufgelöst direkt bestimmt werden, ist es möglich, das Auftreffen des Objekts, bzw. die Kraft, die durch das auftreffende Objekt auf den Auftreffkörper übertragen wird, während des Auftreffens selbst (zeitaufgelöst) genau zu beobachten.

Daher eignet sich das erfindungsgemäße Verfahren insbesondere, um eine zuverlässige Trennung zwischen elastischen und nichtelastischen Objekten vorzunehmen, wobei diese sogar identische Massen haben können. Dies ist besonders relevant, wenn auf die sichere Entsteinung von Steinobst geschlossen werden soll, wo es einen auftreffenden Kern von auftreffendem Fruchtfleisch zu unterscheiden gilt, wobei der Kern hartes, unelastisches Material und das Fruchtfleisch weiches, außerordentlich elastisches Material darstellt.

Da sich anhand des Beispiels des Überprüfens des Entkernvorganges von Steinobstfrüchten das erfindungsgemäße Konzept zum Klassifizieren eines auftreffenden Objekts besonders anschaulich darstellen lässt, wird die nun folgende Beschreibung ohne Beschränkung der Allgemeinheit das erfindungsgemäße Konzept anhand des Entkernens von Steinobstfrüchten illustrieren.

Bei einem speziellen Ausführungsbeispiel der vorliegenden Erfindung wird daher durch geeignete Vorkehrungen, wie z.B. eine geeignete mechanische Anordnung, sichergestellt, dass die aus der Frucht entfernten Fruchtanteile, wie z.B. Kern oder Fruchtsaft, nur entlang eines vorbestimmten Objektweges austreten können.

Tritt also der Kern oder Fruchtfleisch aus einer Frucht aus, wird der Kern oder das Fruchtfleisch auf den Auftreffkörper treffen, der sich im Objektweg befindet, wobei der am ausgelenkten Auftreffkörper bestimmte Beschleunigungsverlauf dazu verwendet wird, zwischen Kern und Fruchtfleisch eindeutig zu unterscheiden.

Somit kann sichergestellt werden, dass der Obststein bei der Entsteinung tatsächlich aus der Frucht entfernt wird, wenn das Auftreffen des Kerns auf dem Sensor detektiert wird. Andernfalls erfolgt eine Fehlermeldung, so dass nichtentsteinte Früchte aus dem Produktstrom entfernt werden können.

Durch Nachweisen des Kerns selbst nach dem Entkernvorgang über die auf einen Auftreffkörper übermittelte Kraft bzw. die daraus bestimmbare Beschleunigung des Auftreffkörpers ergibt sich gegenüber dem Stand der Technik der große Vorteil, dass der Anteil nichtentsteinten Obstes erheblich reduziert werden kann.

Bei einem weiteren Ausführungsbeispiel der vorliegenden Erfindung ist der Auftreffkörper als Federstahlblättchen ausgeführt, das an einer Seite fixiert ist, so dass ein auftreffender Kern bzw. ein auftreffendes Objekt Kraft auf das Federstahlblättchen überträgt, welches ausgelenkt bzw. beschleunigt wird. Nach jedem Auslenken muss ein Beschleunigungssensor bzw. eine Apparatur zum Messen der Beschleunigung in eine Ruhelage zurückgebracht werden, was im Fall des Federstahlblättchens durch die Materialeigenschaften des Federstahls automatisch geschieht.

Bei einem weiteren Ausführungsbeispiel der vorliegenden Erfindung ist der Auftreffkörper an zumindest drei Seiten federnd gelagert, so dass eine Beschleunigungsinformation nicht nur in einer Dimension ermittelt werden kann, da bei nichtzentralem Auftreffen des Objekts der Auftreffkörper auch verkippt werden kann, so dass bei Verwendung eines dreidimensionalen Beschleunigungssensors auch die Richtung der Beschleunigung, also die Auftreffrichtung des Kerns bzw. des Fruchtfleisches rekonstruiert werden kann. Dies kann besonders dann hilfreich sein, wenn beispielsweise ein Kirschkern von einem Stößel nicht zentral getroffen wurde, so dass dieser den Auftreffkörper nicht zentral trifft. Bei nichtzentralem Treffen des Kirschkerns durch den Stößel besteht die Gefahr, den Kirschkern zu spalten, so dass ein Fragment des Kirschkerns in der Kirsche verbleibt. Durch Verwenden des erfindungsgemäß gelagerten Auftreffkörpers kann somit zusätzliche Information darüber gewonnen werden, ob ein Risiko besteht, dass der Kern nicht vollständig entfernt wurde.

Bei einem weiteren Ausführungsbeispiel der vorliegenden Erfindung wird die Beschleunigungsinformation von einer Klassifikationseinrichtung verwendet, um Kerne von Fruchtfleisch zu unterscheiden. Der Verlauf der Beschleunigung ist charakteristisch unter anderem für die Härte, aber auch für die im auftreffenden Körper steckende Energie. Ein hartes auftreffendes Objekt, wie beispielsweise ein Obststein, wird beim Aufprall nicht deformiert und gibt seine Energie direkt an die Zielfläche (den Auftreffkörper) und eine eventuell damit verbundene Rückstellfeder ab. Ein weiches auftreffendes Objekt, wie beispielsweise das Fruchtfleisch, wird beim Aufprall deformiert, es wird die Energie also nur teilweise auf die Zielfläche übertragen. Das Integral über den Beschleunigungsverlauf gibt Aufschluss über die insgesamt auf die Zielfläche übertragene Energie und kann somit als zusätzliches Klassifizierungskriterium herangezogen werden. Daraus lässt sich etwa die Masse eines auftreffenden Objektes bestimmen, wenn dessen Geschwindigkeit bekannt ist. Diese ist beispielsweise im Entkernfall identisch der (bekannten) Geschwindigkeit eines zur Entkernung verwendeten Stössels.

Entscheidend ist jedoch das direkte Auswerten des zeitlichen Beschleunigungsverlaufs, da die gesamte zusätzliche Information durch zeitliches Integrieren der Beschleunigung erhalten werden kann. Durch Betrachten des Kurvenverlaufs der Beschleunigungskurve das Vorhandensein eines harten Körpers unmittelbar ersichtlich, da die Beschleunigung bereits am Beginn der Kurve ihr Maximum erreicht, im Gegensatz zum weichen Körper, wo sich ein kontinuierlicher Beschleunigungsverlauf einstellt.

Bevorzugte Ausführungsbeispiele der vorliegenden Erfindung werden nachfolgend Bezug nehmend auf die beiliegende Zeichnung näher erläutert. Es zeigt:
- Fig. 1: eine schematische Darstellung einer Vorrichtung zur Kontrolle des Erfolges eines Entkernprozesses von Steinobst und dessen Einbindung in den Pro- duktionsprozess;
- Fig. 2: ein Beispiel für eine erfindungsgemäße Vorrich- tung zum Klassifizieren eines Objekts;
- Fig. 3: ein weiteres Beispiel für eine erfindungsgemäße Vorrichtung zum Klassifizieren eines Objekts;
- Fig. 4: ein Beispiel für einen erfindungsgemäßen Auf- treffkörper; und
- Fig. 5: ein weiteres Beispiel für einen erfindungsgemäßen Auftreffkörper.

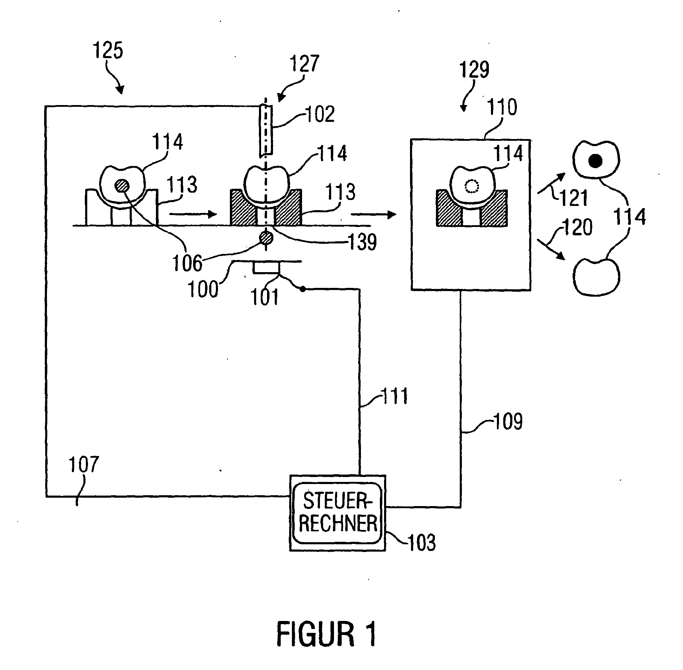

Die Fig. 1 zeigt eine Vorrichtung zur Kontrolle des Erfolges des Entkernvorganges einer Steinobstfrucht gemäß einem Ausführungsbeispiel der vorliegenden Erfindung. Als Beispiel für eine Steinobstfrucht dient eine Kirsche, wobei jedoch die vorliegende Erfindung auch für jede andere Steinobstfrucht verwendbar ist.

Die Vorrichtung zur Kontrolle des Erfolges des Entkernvorganges umfasst einen Auftreffkörper 100, eine Einrichtung zum Erfassen eines zeitlichen Beschleunigungsverlaufs 101, einen Stößel 102 und einen Steuerrechner bzw. eine Klassifizierungseinrichtung 103. Der Steuerrechner 103 ist über eine Signalleitung 107 mit einer Einrichtung zum Betätigen eines Stößels 102 verbunden, sowie über eine weitere Signalleitung 109 mit einer Sortieranlage 110. Die Einrichtung zum Erfassen einer zeitlichen Beschleunigung 101 ist mit dem Steuerrechner 103 über eine Signalleitung 111 verbunden.

Ferner ist eine Halteeinrichtung 113 vorgesehen, in der sich eine Kirsche 114 während des Entkernvorgangs durch den Stößel 102 befindet, und aus der sie durch die Sortieranlage 110 entlang eines ersten Weges 120 oder eines zweiten Weges 121 nach dem Entkernvorgang entfernt und ausgegeben wird. Die Selektion des Weges 120 oder 121 erfolgt je nachdem, ob der Entkernvorgang durch den Steuerrechner 103 als erfolgreich oder nicht erfolgreich angegeben wurde. Dabei zeigt Fig. 1 im Abschnitt 125 den Zeitpunkt vor dem Entkernvorgang, im Abschnitt 127 den Zeitpunkt während des Entkernvorgangs und 129 den Zeitpunkt nach dem Entkernvorgang.

Während des Betriebs wird die Kirsche 114 von der Halteeinrichtung 113 in definierter Position geführt. Beim eigentlichen Entkernvorgang 127 wird die Kirsche 114 von Stößel 102 zentral durchstoßen, sodass durch den Stößel 102 der Kern 106 der Kirsche idealerweise vollständig aus der Kirsche entfernt wird. Die Geometrie der Apparatur ist derart ausgelegt, dass durch sie ein Objektweg festgelegt wird, entlang dessen sich der Kern 106 oder das alternativ oder zusätzlich aus der Frucht herausgelöste Gewebe bewegen muss. Um dies zu erreichen, liegt die Kirsche 114 innerhalb einer Mulde der Halteeinrichtung 113, wobei sich unterhalb der Kirsche eine Bohrung 139 in der Halteeinrichtung 113 befindet, deren Durchmesser klein genug ist, damit die Kirsche 114 nicht durch die Bohrung 139 fallen kann. Andererseits muss der Durchmesser der Bohrung 139 geringfügig größer als der Durchmesser des zylindrischen Stößels 102 sein, damit dieser in die Bohrung 139 von Halteeinrichtung 113 eintreten kann. Beim Entkernvorgang durchdringt der Stößel 102 die Kirsche 114 beispielsweise vollständig und tritt ebenfalls durch die Bohrung 139 hindurch, sodass das aus der Kirsche entfernte Gewebe zwangsweise unterhalb der Halteeinrichtung 113 aus der Bohrung 139 herausbefördert wird. Das entfernte Gewebe fällt unter dem Einfluss der Schwerkraft nach unten.

Um das Messergebnis der Einrichtung zum Erfassen eines zeitlichen Beschleunigungsverlaufs 101 zeitlich mit einem Entkernvorgang synchronisieren zu können, ist der Steuerrechner 103 über die Signalleitung 107 mit dem Stößel 102, der das Entkernen vornimmt, gekoppelt, wobei der Steuerrechner entweder aktiv den Beginn des Entkernprozesses steuern kann oder von dem Stößel 102 ein Signal als Mitteilung darüber enthält, wann der zu überwachende Entkernvorgang stattfindet.

Abhängig von Erfolg oder Misserfolg des Entkernvorganges kann die Steuereinrichtung 103 über die Signalleitung 109 die Sortieranlage 110 derart steuern, dass vollständig entkernte Steinobstfrüchte von nicht vollständig entkernten Steinobstfrüchten getrennt werden, indem die vollständig entkernten Früchte den ersten Weg 120 einschlagen, während nicht vollständig entkernte Früchte, also auch solche, die noch Teile eines Kerns beinhalten, dem zweiten Weg 121 folgen.

Wie es Fig. 1 zu entnehmen ist, ist der Auftreffkörper 100 mit der Einrichtung zum Erfassen eines zeitlichen Beschleunigungsverlaufs 101 derart geometrisch angeordnet, dass er sich in einem Objektweg befindet, entlang dessen sich der austretende Kirschkern bzw. das austretende Fruchtfleisch bewegt. Zusätzlich kann hier vorgesehen sein, dass der Objektweg durch weitere bauliche Maßnahmen geometrisch eingeschränkt ist. Dies können beispielsweise Prallwände bzw. eine zylindrische Einfassung des Auftreffkörpers sein, die mit der Unterseite der Halteeinrichtung 113 abschließt, so dass sich die Bohrung 139 innerhalb der zylindrischen-Umrandung befindet.

Um einen Kern von Fruchtfleisch zu unterscheiden, ist dabei beispielsweise eine zeitliche Auflösung des gemessenen Beschleunigungsverlaufs ausreichend.

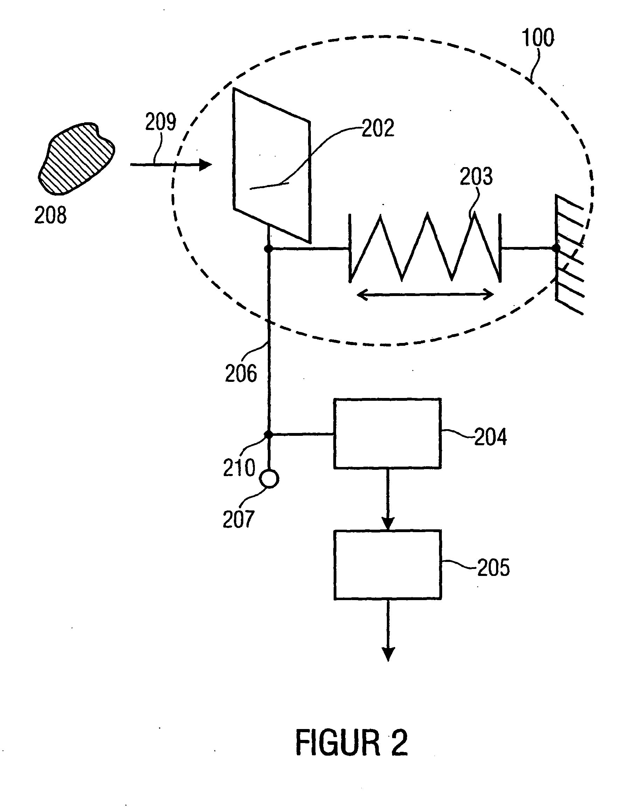

Fig. 2 zeigt ein weiteres Ausführungsbeispiel einer erfindungsgemäßen Vorrichtung zum Klassifizieren eines entlang eines Objektweges auftreffenden Objekts.

Fig. 2 zeigt eine Zielfläche 202, eine Feder 203, einen Beschleunigungssensor 204 sowie eine Auswerteeinheit 205. Die Zielfläche 202 ist über einen Steg 206 mit einem Lager 207 verbunden, an dem der Steg 206 einseitig drehend gelagert ist. Die Feder 203 stellt sicher, dass der Steg, wenn er aus seiner Ruhelage bewegt wird, durch die Federkraft zurückgestellt wird, so dass sich die Zielfläche 202 nach Auftreffen eines Objekts 208 wiederum in Ruhelage befindet.

Das Objekt 208 bewegt sich entlang eines Objektweges 209 und trifft somit auf die Zielfläche 202, die aus ihrer Ruhelage ausgelenkt wird, um durch die Feder 203 für die nächste Messung wieder in die Ruhelage zurückgebracht zu werden. Während der Auslenkung nimmt der Beschleunigungssensor 204 kontinuierlich den Verlauf der Beschleunigung der Zielfläche auf. Dieser Verlauf wird von der Auswerteeinheit 205 bewertet und zur Klassifikation des auftreffenden Objekts verwendet. In dem in Fig. 2 gezeigten Ausführungsbeispiel bilden die Feder 203 und die Zielfläche 202 also einen Auftreffkörper 100 im Sinne des in Fig. 1 gezeigten Auftreffkörpers.

Bei der Beschreibung der Figuren sowie im weiteren Verlauf der Anmeldung sind dieselben funktionalen Komponenten mit denselben Bezugszeichen versehen, so dass deren Beschreibung innerhalb der Figurenbeschreibungen der einzelnen Ausführungsbeispiele wechselseitig aufeinander anwendbar ist.

Das in Fig. 2 gezeigte Ausführungsbeispiel verwendet eine mechanisch besonders robuste Konstruktion, um die erfindungsgemäße Vorrichtung zum Klassifizieren eines entlang eines Objektweges auftreffenden Objektes zu realisieren. Ein Anlenkpunkt 210, der den Kraftschluss zwischen dem Beschleunigungssensor 204 und dem Ausleger 206 herstellt, kann darüber hinaus problemangepasst beliebig variiert werden, um die für die Problemstellung ideale Empfindlichkeit des Beschleunigungssensors 204 zu erzielen.

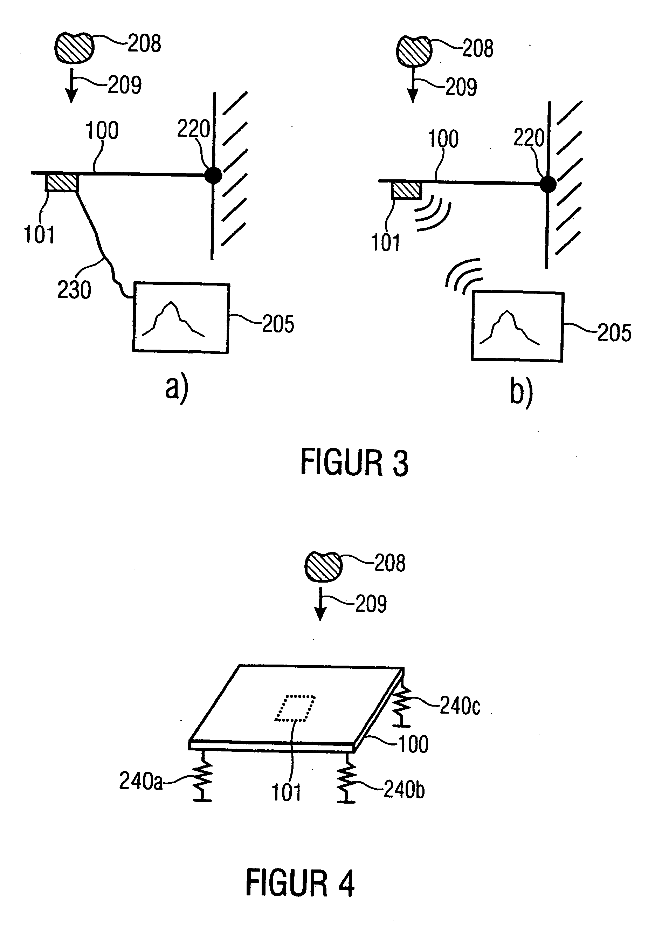

Fig. 3a zeigt ein weiteres Ausführungsbeispiel einer erfindungsgemäßen Vorrichtung zum Klassifizieren eines Objekts, wobei der Auftreffkörper 100 durch ein Federstahlblättchen gebildet wird, welches einseitig an einer Fixierung 220 fixiert ist, so dass bei Auftreffen eines Objekts 208, welches sich entlang eines Objektweges 209 bewegt, das Federstahlblättchen aus seiner Ruhelage ausgelenkt wird. Die Einrichtung zum Erfassen eines zeitlichen Beschleunigungsverlaufs 101 erfasst die Beschleunigung des Federstahlblättchens 100 am Ort der Erfassungseinrichtung 101 und überträgt diese an eine Klassifikationseinrichtung 205. Aufgrund des charakteristischen Beschleunigungsverlaufs kann die Klassifikationseinrichtung das Objekt 208, das das Federstahlblättchen 100 tritt, klassifizieren, um so beispielsweise einen Kern von Fruchtfleisch zu unterscheiden.

Da es, wie bereits mehrfach erwähnt, erst durch die zeitaufgelöste Beschleunigungsmessung möglich wird, Kerne von Fruchtfleisch bzw. harte von elastischen Objekten zuverlässig zu unterscheiden, ist erfindungsgemäß besonders darauf zu achten, dass die Messung nicht durch externe Krafteinflüsse verfälscht wird.

Daher ist anhand von Fig. 3b eine weitere Ausführungsform der Vorrichtung zum Klassifizieren eines auftreffenden Objekts dargestellt, in der die Einrichtung zum Erfassen eines zeitlichen Beschleunigungsverlaufs 101 mit der Klassifikationseinrichtung 205 mittels einer drahtlosen Schnittstelle verbunden ist, um einen eventuellen zusätzlichen Krafteinfluss durch eine drahtgebundene Schnittstelle 230, wie sie in Fig. 3a gezeigt ist, zu unterbinden. Eine solche drahtlose Schnittstelle kann dabei beispielsweise eine Bluetooth-, WLAN-, Infrarot- oder Laserschnittstelle sein oder jedwede andere Möglichkeit der drahtlosen Kommunikation. Auch RFID-Techniken sind hier besonders sinnvoll einsetzbar, in denen die Einrichtung zum Erfassen des zeitlichen Beschleunigungsverlaufs 101 keine eigene Energiequelle benötigt, sondern aus dem Feld der RFID-Verbindung gespeist wird, so dass sich das Gewicht der Einrichtung zum Erfassen eines zeitlichen Beschleunigungsverlaufs 101 durch etwaige Akkumulatoren oder andere Energieträger nicht unnötig erhöht.

Fig. 4 zeigt ein weiteres Ausführungsbeispiel der vorliegenden Erfindung, bei dem der Auftreffkörper 100 durch zumindest drei an den Ecken des Auftreffkörpers 100 angeordnete Federn 240a - 240c in seiner Ruhelage gehalten wird. Die Einrichtung zum Erfassen des zeitlichen Beschleunigungsverlaufs 101 ist dabei vorzugsweise an der Unterseite des Auftreffkörpers 100 angeordnet, um eine mechanische Beschädigung bzw. Verschmutzung derselben zu vermeiden. In einer Variation des in Fig. 4 gezeigten Ausführungsbeispiels ist die Federcharakteristik der Federn 240a - 240c progressiv, d. h. zu Beginn der Auslenkung ist die Rückstellkraft geringer als gegen Ende der durch die Federlänge begrenzten Maximalauslenkung. Da die Rückstellkraft der Feder eine zwangskraftfreie Messung der Beschleunigung behindert, ist eine solche Ausgestaltung der Federn günstig, um das Messergebnis so wenig wie möglich zu verfälschen. Die Klassifikationseinrichtung ist in Fig. 4 nicht gezeigt, bezüglich deren Ausgestaltung bzw. der Verbindung derselben mit der Einrichtung zum Erfassen des zeitlichen Beschleunigungsverlaufs 101 sei an dieser Stelle auf die Ausführungen zu den anderen Figuren, insbesondere zu Fig. 3a und 3b, verwiesen.

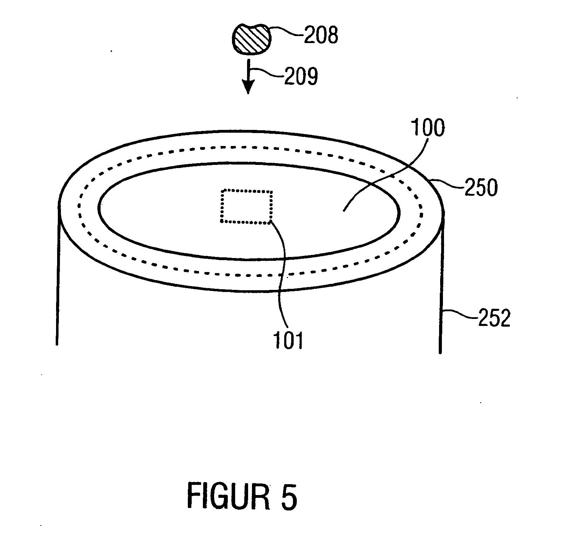

Fig. 5 zeigt ein weiteres Ausführungsbeispiel einer erfindungsgemäßen Vorrichtung zum Klassifizieren eines entlang eines Objektweges 209 auftreffenden Objektes 208, wobei hier die Einrichtung zum Erfassen eines zeitlichen Beschleunigungsverlaufs 101 an der Unterseite einer als Auftreffkörper 100 dienenden Membran angeordnet ist, die, wie beispielsweise bei Lautsprechern üblich, konzentrisch mittels einer Sicke 250 an einem starren Träger 252 montiert ist. Auch hier ist der Einfachheit halber die Klassifikationseinrichtung nicht gezeigt, bezüglich der Ausführungsformen derselben sei auch hier auf die Ausführung zu den übrigen Figuren verwiesen.

Wie bereits erwähnt, ist das Entkernen von Steinobst hier nur exemplarisch zur Illustration des erfindungsgemäßen Konzeptes zu verstehen. Erfindungsgemäß können auch die Eigenschaften beliebiger anderer Körper bestimmt werden. Beispielsweise kann überprüft werden, ob ein Fußball prall aufgepumpt ist oder ob dieser so wenig Innendruck aufweist, dass er sich beim Auftreffen auf den Auftreffkörper signifikant verformt.

Im am Beispiel von Fig. 1 gezeigten Szenario zur Überprüfung des erfolgreichen Entkernens von Steinobst wird der Kern aus der Frucht mittels eines Stößels entfernt. Die vorliegende Erfindung ist jedoch auf die Art des Entfernens des Kernes nicht eingeschränkt. Es muss lediglich sichergestellt sein, dass das zu klassifizierende Objekt auf den Auftreffkörper trifft.

Auch ist die vorliegende Erfindung nicht auf die Überprüfung von Kirschkernen, wie es in Fig. 1 gezeigt ist, beschränkt. Vielmehr können neben beliebigen Objekten insbesondere auch Kerne von Pflaumen, Pfirsichen oder Aprikosen von Fruchtfleisch derselben Früchte unterschieden werden, um den Erfolg eines Entkernvorganges zu überprüfen.

Die Art und Weise, wie die Beschleunigung des Auftreffkörpers bestimmt wird, ist in keiner Weise festgelegt. In einem einfachen Ausführungsbeispiel kann ein eindimensional wirksamer Beschleunigungssensor verwendet werden, der eine Beschleunigung lediglich in einer Richtung, die bevorzugt parallel zur Auftreffrichtung des Objekts ist, bestimmt. Alternativ kann auch ein dreidimensional messender Beschleunigungssensor verwendet werden, um zusätzliche Informationen über die Auftreffrichtung des zu klassifizierenden Objekts zu gewinnen. Dies kann, wie bereits beschrieben, bei Überprüfung von Steinobstentkernung sinnvoll sein, um einen Rückschluss darauf zu ziehen, ob der Kern vollständig oder eventuell nur teilweise aus der Frucht entfernt wurde. Zur einfachen Unterscheidung, ob besonders Harte oder besonders Weiche Objekte den Auftreffkörper getroffen haben, ist in einem einfachen Ausführungsbeispiel der vorliegenden Erfindung die Bestimmung der maximal auftretenden Beschleunigung ausreichend. Dies hat den Vorteil, dass nur geringe Rechenkapazität erforderlich ist und das Ergebnis unmittelbar nach Auftreffen des Objekts zur Verfügung steht. In einer erweiterten Ausführungsform wird der gesamte Beschleunigungskurvenverlauf verwendet, um eine Klassifizierung zu erreichen. Dabei kann insbesondere durch die Art des Anstiegs der Beschleunigung auf die Härte bzw. Viskosität des Objekts geschlossen werden, da ein rascher Anstieg auf einen harten Gegenstand hinweist.

Bei einem weiteren Ausführungsbeispiel kann dabei beispielsweise bestimmt werden, in welcher Zeitspanne die Maximalbeschleunigung erreicht wird, um so annähernd unabhängig von der Masse des auftreffenden Objekts sicher und zuverlässig eine Aussage über die Härte des Objekts treffen zu können.

Abhängig von den Gegebenheiten kann das erfindungsgemäße Verfahren zum Überprüfen des Erfolges eines Entkernvorganges an Steinobstfrüchten in Hardware oder in Software implementiert werden. Die Implementation kann auf einem digitalen Speichermedium, insbesondere einer Diskette oder CD mit elektronisch auslesbaren Steuersignalen erfolgen, die so mit einem programmierbaren Computersystem zusammenwirken können, dass das erfindungsgemäße Verfahren zum überprüfen des Erfolges eines Entkernvorganges ausgeführt wird. Allgemein besteht die Erfindung somit auch in einem Computer-Programm-Produkt mit einem auf einem maschinenlesbaren Träger gespeicherten Programmcode zur Durchführung des erfindungsgemäßen Verfahrens, wenn das Computer-Programm-Produkt auf einem Rechner abläuft. In anderen Worten ausgedrückt kann die Erfindung somit als ein Computer-Programm mit einem Programmcode zur Durchführung des Verfahrens realisiert werden, wenn das Computer-Programm auf einem Computer abläuft.

Abschließend sei noch erwähnt, dass die Vorrichtung zum Klassifizieren eines entlang eines Objektweges 209 auftreffenden Objektes 208 nicht nur, wie beschrieben, einen Auftreffkörper 100, der in dem Objektweg 209 angeordnet ist, eine Einrichtung zum Erfassen eines zeitlichen Beschleunigungsverlaufs 101 des Auftreffkörpers 100 auf ein Auftreffen eines Objekts hin und eine Klassifikationseinrichtung 103/205 zum, basierend auf dem zeitlichen Beschleunigungsverlauf, Klassifizieren des Objekts aufweisen kann, sondern zusätzlich kann dabei die Einrichtung zum Erfassen eines zeitlichen Beschleunigungsverlaufs so ausgebildet sein, dass der Beschleunigungsverlauf in einem analogen Signal dargestellt ist, und weiterhin zusätzlich beispielsweise so, um das analoge Signal zu digitalisieren.

## Patentansprüche

1. Vorrichtung zum Klassifizieren eines entlang eines Objektweges (209) auftreffenden Objektes (208), mit folgenden Merkmalen:
einem Auftreffkörper (100), der in dem Objektweg (209) angeordnet ist, um bei einem Auftreffen des Objektes teilweise aus einer Ruhelage ausgelenkt zu werden;
einen Beschleunigungssensor zum Erfassen eines zeitlichen Beschleunigungsverlaufs (101) des Auftreffkörpers (100) während der Auslenkung aus der Ruhelage auf das Auftreffen des Objekts hin, wobei der Beschleunigungssensor als diskretes Bauteil ausgebildet ist, um den Beschleunigungsverlauf ohne mechanische Referenz zu erfassen; und
einer Klassifikationseinrichtung (103; 205) zum, basierend auf dem zeitlichen Beschleunigungsverlauf, Klassifizieren des Objekts.

2. Vorrichtung gemäß Anspruch 1, bei der der Beschleunigungssensor ausgebildet ist, um den Beschleunigungsverlauf dreidimensional zu erfassen.

3. Vorrichtung gemäß Anspruch 2, bei der der Beschleunigungssensor ausgebildet ist, um eine Beschleunigung eindimensional in einer Richtung, die im wesentlichen parallel zum Objektweg ist, zu erfassen.

4. Vorrichtung gemäß einem der vorhergehenden Ansprüche, die ferner eine Rückstelleinrichtung aufweist, um den Auftreffkörper nach dem Auslenken in die Ruhelage zurückzuführen.

5. Vorrichtung gemäß Anspruch 4, bei der die Rückstelleinrichtung ausgebildet ist, eine progressiv wirkende Rückstellkraft auf den Auftreffkörper auszuüben.

6. Vorrichtung gemäß einem der vorhergehenden Ansprüche, bei der der Auftreffkörper ein an einer Seite fixiertes Federstahlblättchen ist.

7. Vorrichtung gemäß einem der Ansprüche 1 bis 5, bei der der Auftreffkörper eine Auftrefffläche aufweist, die an zumindest drei Punkten federnd gelagert ist, so dass bei nichtzentralem Auftreffen des Objekts der Auftreffkörper auch verkippt werden kann.

8. Vorrichtung gemäß Anspruch 1, bei der der Auftreffkörper eine Auftrefffläche aufweist, die mittels einer Feder in einer Ruhelage gehalten wird.

9. Vorrichtung gemäß einem der vorhergehenden Ansprüche, bei der die Klassifikationseinrichtung ausgebildet ist, eine Klassifikation aufgrund eines beobachteten Überschreitens einer vorbestimmten Beschleunigungsschwelle durchzuführen.

10. Vorrichtung gemäß einem der vorhergehenden Ansprüche, bei der die Klassifikationseinrichtung ausgebildet ist, eine Klassifikation des Objekts aufgrund eines zeitlichen Beschleunigungsverlaufs mit einer Zeitauflösung von weniger als zehn Millisekunden durchzuführen.

11. Vorrichtung gemäß einem der vorhergehenden Ansprüche, bei der die Klassifikationseinrichtung ausgebildet ist, um die Klassifikation des Objekts auf Basis der maximal auftretenden Beschleunigung des zeitlichen Beschleunigungsverlaufs oder einer Zeitspanne, in der die maximal auftretende Beschleunigung erreicht wird, durchzuführen.

12. Vorrichtung gemäß einem der vorhergehenden Ansprüche, bei der das Objekt ein aus einem Steinobst entferntes Gewebe ist, und bei der die Klassifikationseinrichtung ausgebildet ist, um das Objekt dahin gehend zu klassifizieren, ob ein Kern vollständig im Gewebe enthalten ist.

13. Vorrichtung gemäß einem der vorhergehenden Ansprüche, bei der der Beschleunigungssensor zum Erfassen eines zeitlichen Beschleunigungsverlaufs (101) ausgebildet ist, um einen erfassten Beschleunigungsverlauf über eine RFID-Verbindung zu der Klassifikationseinrichtung zu übertragen, und aus dem Feld der RFID-Verbindung gespeist zu werden, so dass der Beschleunigungssensor keine eigene Energiequelle benötigt.

14. Vorrichtung gemäß einem der vorhergehenden Ansprüche, bei der der Beschleunigungssensor (101) als Chip ausgebildet, der den erfassten Beschleunigungsverlaufs drahtlos kommunizieren kann.

15. Vorrichtung gemäß einem der vorhergehenden Ansprüche, bei der der Beschleunigungssensor an einer dem Objektweg abgewandten Unterseite des Autreffkörpers angeordnet ist.

16. Verfahren zum Klassifizieren eines entlang eines Objektweges auftreffenden Objektes, mit folgenden Schritten:
Erfassen, mittels eines Beschleunigungssensors, eines zeitlichen Beschleunigungsverlaufs eines Auftreffkörpers, der in dem Objektweg angeordnet ist, während einer Auslenkung des Auftreffkörpers aus einer Ruhelage auf ein Auftreffen des Objekts hin; und Klassifizieren des Objekts, basierend auf dem zeitlichen Beschleunigungsverlauf.

17. Entkernvorrichtung für Steinobst, mit folgenden Merkmalen:
einer Entkerneinrichtung zum entfernen von Gewebe aus dem Steinobst entlang eines Objektweges (209);
einem Auftreffkörper (100), der in dem Objektweg (209) angeordnet ist, um bei einem Auftreffen des Gewebes teilweise aus einer Ruhelage ausgelenkt zu werden;
einer Beschleunigungssensor zum Erfassen eines zeitlichen Beschleunigungsverlaufs (101) des Auftreffkörpers (100) während der Auslenkung aus der Ruhelage auf das Auftreffen des Gewebes hin, wobei der Beschleunigungssensor als diskretes Bauteil ausgebildet ist, um den Beschleunigungsverlauf ohne mechanische Referenz zu erfassen; und
einer Klassifikationseinrichtung (103; 205) zum, basierend auf dem zeitlichen Beschleunigungsverlauf, erkennen eines Kernes in dem Gewebe.

## Claims

1. An apparatus for classifying an object (208) impinging along an object path (209), the apparatus comprising:
an impingement body (100) arranged within the object path (209), to be partly deflected from a resting position upon impingement of the object;
an acceleration sensor for sensing a chronological acceleration profile (101) of the impingement body (100) upon impingement of an object during the deflection from the resting position, the acceleration sensor being implemented as a discrete component so as to sense the acceleration profile without any mechanical reference; and
a classification means (103; 205) for classifying the object on the basis of the chronological acceleration profile.

2. The apparatus as claimed in claim 1, wherein the acceleration sensor is implemented to three-dimensionally sense the acceleration profile.

3. The apparatus as claimed in claim 2, wherein the acceleration sensor is implemented to unidimensionally sense an acceleration in a direction which is essentially parallel to the object path.

4. The apparatus as claimed in any of the previous claims, further comprising a restoring means so as to return the impingement body to the resting position after it has been deflected.

5. The apparatus as claimed in claim 4, wherein the restoring means is implemented to exert a restoring force, which acts progressively, on the impingement body.

6. The apparatus as claimed in any of the previous claims, wherein the impingement body is a spring-steel lamella fixed on one side.

7. The apparatus as claimed in one of claims 1 to 5, wherein the impingement body comprises an impingement area which is spring-mounted at at least three points, so that if the object impinges in a non-central manner, the impingement body may also be tilted.

8. The apparatus as claimed in claim 1, wherein the impingement body comprises an impingement area which is held in a resting position by a spring.

9. The apparatus as claimed in any of the previous claims, wherein the classification means is implemented to perform a classification on the basis of an observed overshoot of a predetermined acceleration threshold.

10. The apparatus as claimed in any of the previous claims, wherein the classification means is implemented to perform a classification of the object on the basis of a chronological acceleration profile with a time resolution of less than ten milliseconds.

11. The apparatus as claimed in any of the previous claims, wherein the classification means is implemented to perform the classification of the object on the basis of maximally occurring acceleration of the chronological acceleration profile or a period of time within which the maximum acceleration is achieved.

12. The apparatus as claimed in any of the previous claims, wherein the object is a tissue removed from a piece of stone fruit, and wherein the classification means is implemented to classify the object in terms of whether a stone is entirely contained within the tissue.

13. The apparatus as claimed in any of the previous claims, wherein the acceleration sensor for sensing a chronological acceleration profile (101) is implemented to transmit a sensed acceleration profile via a RFID connection to the classification means, and to be supplied from the field of the RFID connection so that the acceleration sensor requires no energy source of its own.

14. The apparatus as claimed in any of the previous claims, wherein the acceleration sensor (101) is implemented as a chip which may communicate the sensed acceleration profile in a wireless manner.

15. The apparatus as claimed in any of the previous claims, wherein the acceleration sensor is arranged on an underside of the impingement body facing away from the object path.

16. A method of classifying an object impinging along an object path, the method comprising:
sensing, by means of an acceleration sensor, a chronological acceleration profile of an impingement body arranged in the object path upon impingement of an object during a deflection of the impingement body from the resting position,; and
classifying the object on the basis of the chronological acceleration profile.

17. A stoning apparatus for stone fruit, comprising:
a stoning means for removing tissue along an object path (209) from the stone fruit;
an impingement body (100) arranged within the object path (209) to be partly deflected from a resting position upon impingement of the tissue;
an acceleration sensor for sensing a chronological acceleration profile (101) of the impingement body (100) upon impingement of the tissue during the deflection from the resting position, the acceleration sensor being implemented as a discrete component so as to sense the acceleration profile without any mechanical reference; and
a classification means (103; 205) for recognizing a stone within the tissue on the basis of the chronological acceleration profile.

## Revendications

1. Dispositif de classification d'objet d'incidence (208) le long d'un trajet d'objet (209), aux caractéristiques suivantes :
un corps d'incidence (100) qui est disposé dans le trajet d'objet (209), pour être dévié partiellement hors d'une position de repos en cas d'incidence de l'objet ;
un capteur d'accélération destiné à saisir une évolution d'accélération dans le temps (101) du corps d'incidence (100) pendant la déviation hors de la position de repos après l'incidence de l'objet, le capteur d'accélération étant réalisé sous forme de composant discret, pour saisir l'évolution d'accélération sans référence mécanique ; et
un moyen de classification (103; 205) destiné à classifier l'objet sur base de l'évolution d'accélération dans le temps.

2. Dispositif selon la revendication 1, dans lequel le capteur d'accélération est réalisé pour saisir l'évolution d'accélération de manière tridimensionnelle.

3. Dispositif selon la revendication 2, dans lequel le capteur d'accélération est réalisé pour saisir une accélération de manière unidimensionnelle dans une direction qui est sensiblement parallèle au trajet d'objet.

4. Dispositif selon l'une des revendications précédentes, présentant par ailleurs un moyen de rappel pour ramener le corps d'incidence en position de repos après la déviation.

5. Dispositif selon la revendication 4, dans lequel le moyen de rappel est réalisé pour exercer une force de rappel agissant progressivement sur le corps d'incidence.

6. Dispositif selon l'une des revendications précédentes, dans lequel le corps d'incidence est une lame d'acier élastique fixée à un côté.

7. Dispositif selon l'une des revendications 1 à 5, dans lequel le corps d'incidence présente une face d'incidence qui est montée de manière élastique en au moins trois points, de sorte que, en cas d'incidence non centrale de l'objet, le corps d'incidence puisse également être culbuté.

8. Dispositif selon la revendication 1, dans lequel le corps d'incidence présente une face d'incidence qui est maintenue en position de repos au moyen d'un ressort.

9. Dispositif selon l'une des revendications précédentes, dans lequel le moyen de classification est réalisé pour effectuer une classification sur base d'un dépassement observé d'un seuil d'accélération prédéterminé.

10. Dispositif selon l'une des revendications précédentes, dans lequel le moyen de classification est réalisé pour effectuer une classification de l'objet sur base d'une évolution d'accélération dans le temps avec une résolution temporelle de moins de dix millisecondes.

11. Dispositif selon l'une des revendications précédentes, dans lequel le moyen de classification est réalisé pour effectuer la classification de l'objet sur base de l'accélération maximale se produisant de l'évolution d'accélération dans le temps ou d'un laps de temps dans lequel est atteinte l'accélération maximale se produisant.

12. Dispositif selon l'une des revendications précédentes, dans lequel l'objet est un tissu enlevé d'un fruit à noyau, et dans lequel le moyen de classification est réalisé pour classifier l'objet selon que le noyau est ou non contenu totalement dans le tissu.

13. Dispositif selon l'une des revendications précédentes, dans lequel le capteur d'accélération destiné à saisir une évolution d'accélération dans le temps (101) est réalisé pour transmettre une évolution d'accélération saisie via une connexion RFID au moyen de classification, et pour être alimenté à partir du champ de la connexion RFID, de sorte que le capteur d'accélération ne requière pas de source d'énergie qui lui est propre.

14. Dispositif selon l'une des revendications précédentes, dans lequel le capteur d'accélération (101) est réalisé sous forme de puce qui peut communiquer sans fil l'évolution d'accélération saisie.

15. Dispositif selon l'une des revendications précédentes, dans lequel le capteur d'accélération est disposé sur une face inférieure du corps d'incidence éloignée du trajet d'objet.

16. Procédé pour classifier un objet d'incidence le long d'un trajet d'objet, aux étapes suivantes consistant à:
saisir, au moyen d'un capteur d'accélération, une évolution d'accélération dans le temps d'un corps d'incidence qui est disposé dans le trajectoire d'objet, pendant une déviation du corps d'incidence hors de la position de repos après l'incidence de l'objet; et
classifier l'objet sur base de l'évolution d'accélération dans le temps.

17. Dispositif de dénoyautage pour fruit à noyau, aux caractéristiques suivantes:
un moyen de dénoyautage destiné à enlever le tissu du fruit à noyau le long d'un trajet d'objet (209) ;
un corps d'incidence (100) qui est disposé dans le trajet d'objet (209), pour être dévié, en cas d'incidence du tissu, partiellement hors d'une position de repos ;
un capteur d'accélération destiné à saisir une évolution d'accélération dans le temps (101) du corps d'incidence (100) pendant une déviation du corps d'incidence hors de la position de repos après l'incidence de l'objet, le capteur d'accélération étant réalisé comme composant discret, pour saisir l'évolution d'accélération sans référence mécanique ; et
un moyen de classification (103; 205) destiné à identifier, sur base de l'évolution d'accélération dans le temps, un noyau dans le tissu.
